(19) Europäisches Patentamt
European Patent Office
Office européen des brevets

(11) Veröffentlichungsnummer: **0 087 657**
**B1**

(12) # EUROPÄISCHE PATENTSCHRIFT

(45) Veröffentlichungstag der Patentschrift: **14.05.86**

(51) Int. Cl.4: **C 07 D 209/28**, C 07 D 403/06

(21) Anmeldenummer: **83101355.2**

(22) Anmeldetag: **12.02.83**

(54) **Verfahren zur Herstellung von 1-(4-Chlorbenzoyl)-5-methoxy-2-methyl-3-indolacetoxyessigsäure.**

(30) Priorität: **26.02.82 DE 3206887**

(43) Veröffentlichungstag der Anmeldung:
**07.09.83 Patentblatt 83/36**

(45) Bekanntmachung des Hinweises auf die Patenterteilung:
**14.05.86 Patentblatt 86/20**

(84) Benannte Vertragsstaaten:
**AT BE CH DE FR GB IT LI LU NL SE**

(56) Entgegenhaltungen:
**DE - A - 2 740 854**

**CHEMICAL ABSTRACTS, Band 88, Nr. 7, 13. Februar 1978, Seite 373, Nr. 62247m, Columbus, Ohio, USA, L. FISNEROVA et al.: "Pharmacologically interesting indomethacin derivates. I."**
**CHEMICAL ABSTRACTS, Band 90, Nr. 10, 5. März 1979, Nr. 87267x, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG, Berliner Strasse 156, D-5000 Köln 80 (DE)**

(72) Erfinder: **Boltze, Karl-Heinz, Dr., Ackerstrasse 8, D-5231 Borod (DE)**

(74) Vertreter: **Jesse, Ralf-Rüdiger, Dr. et al, Bayer AG Konzernverwaltung RP Patentabteilung, D-5090 Leverkusen 1 Bayerwerk (DE)**

EP 0 087 657 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung der bekannten 1-(4-Chlorbenzoyl)-5-methoxy-2-methyl-3-indolacetoxyessigsäure (Acemetacin).

Für die Herstellung dieser bekannten Verbindung sind bereits eine Reihe von Verfahren bekannt geworden, vgl. z. B. DE-OS 234651, DE-OS 2257867 und DE-OS 2943125.

Bei den bekannten Verfahren wurde die Carboxygruppe zunächst durch einen Benzylrest geschützt, so dass in einem letzten Reaktionsschritt eine katalytische Hydrierung des Benzylesters gemäss folgendem Reaktionsschema durchgeführt werden musste.

Reaktionsschema

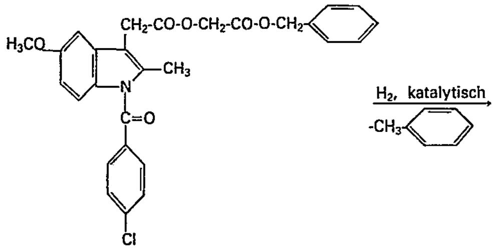


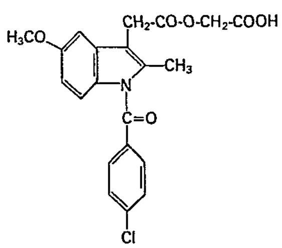

I

Bei dieser Abspaltung des Benzylrestes entsteht als Nebenprodukt stets 1-Benzoyl-5-methoxy-2-methyl-3-indolacetoxyessigsäure, nachstehend Des-Chlor-Verbindung genannt. Diese unerwünschte Verunreinigung, die durch Abspaltung des Chlors aus dem Benzolring des 4-Chlorbenzoylrestes bis zu einer Menge von 0,5% entsteht, muss in aufwendigen Reinigungsschritten anschliessend entfernt werden, was mit Ausbeuteverlusten verbunden ist.

CA 88, 62247m beschreibt die Umsetzung beispielsweise eines Anhydrids, Chlorids oder Imidazolids des Indometacins mit einer nicht näher beschriebenen Alkylverbindung zu Alkylestern des Indometacins.

CA 90, 87267× zeigt die Umsetzung von Indometacin mit Thionylchlorid in Ethylether/Pyridin zu dem Säurechlorid, das dann mit Glykolsäure Acemetacin ergibt.

Es wurde ein Verfahren zur Herstellung von 1-(4-Chlorbenzoyl)-5-methoxy-2-methyl-3-indolacetoxyessigsäure gefunden, das dadurch gekennzeichnet ist, dass man das Derivat der Indolcarbonsäure der Formel

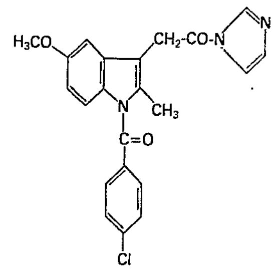

(II),

mit Verbindungen der Formel

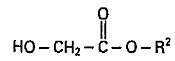

(III),

in welcher
$R^2$ für Wasserstoff oder Ammonium steht,
in Gegenwart von inerten organischen Lösungsmitteln unter absolut wasserfreien Bedingungen in einem Temperaturbereich von −10 bis 80°C umsetzt.

Nach dem erfindungsgemässen Verfahren hergestellte 1-(4-Chlorbenzoyl)-5-methoxy-2-methyl-3-indolacetoxyessigsäure ist überraschenderweise frei von Deschlorverbindungen.

Das erfindungsgemässe Verfahren kann in Gegenwart von inerten organischen Lösungsmitteln wie z. B. Ether, Diethylether, Diisopropylether, Dioxan, Tetrahydrofuran, 1,2-Dimethoxyethan, chlorierte Kohlenwasserstoffe, Methylenchlorid, Chloroform, Dichlorethan, substituierte Amide, Dimethylformamid, N-Methylpyrrolidon, Aromaten, Toluol, Xylol, Ketone, Aceton, Methylethylketon (Butanon-2-) durchgeführt werden.

Die Umsetzung wird im Temperaturbereich von −10 bis 80°C, bevorzugt bei −10 bis 50°C, besonders bevorzugt bei −5 bis 20°C durchgeführt.

Das erfindungsgemässe Verfahren kann durch das folgende Formelschema erläutert werden:

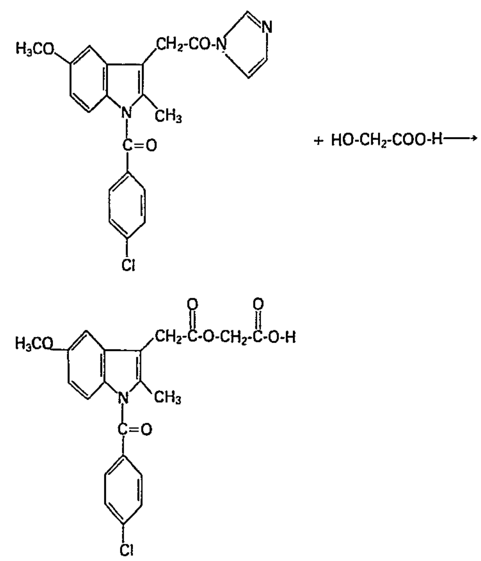

Bevorzugte Verbindung der Formel III ist das Ammoniumsalz der Glykolsäure.

Die entstandenen Ammoniumverbindungen werden anschliessend durch Behandlung mit Säuren, in einfacher Weise in das Endprodukt I (Acemetacin) überführt.

Es war überraschend, dass nach diesen Verfahren die 1-(4-Chlorbenzoyl)-5-methoxy-2-methyl-3-indolacetoxyessigsäure in so reiner Form, d. h. frei von der störenden Des-Chlor-Verbindung, und in Ausbeuten von 60 bis 70% der Theorie entsteht.

Die als Ausgangsstoffe verwendeten Verbindungen der Formel II und III sind bekannt oder werden nach bekannten Verfahren hergestellt.

Die nach den erfindungsgemässen Verfahren hergestellte Endverbindung I ist ein wertvoller pharmazeutischer Wirkstoff mit antiphlogistischer Wirkung, vgl. z. B. DE-PS 2234651.

Im vorliegenden Verfahren erfolgt die Aktivierung der Carboxylgruppe der Formel II ($R^1$ = OH) durch Bildung des Imidazolidderivates, indem die Indolcarbonsäure mit Carbonyldiimidazol nach bekanntem Verfahren umgesetzt wird (vgl. Reaktionsschema). Dieses Zwischenprodukt ist sehr reaktiv und wird in der Regel nach seiner Entstehung sofort weiter umgesetzt. Im vorliegenden Fall führt die Reaktion mit Triethylammoniumglykolat zu dem Ammoniumsalz von I, dessen kationische Komponente im Zuge der Aufarbeitung gegen das Diisopropylammoniumkation ausgetauscht wird. Es bildet sich eine kristalline farblose Substanz, die isoliert und getrocknet wird.

Zur Überführung des Diisopropylammoniumsalzes von I in die freie Säure stellt man eine Lösung in wässrigem Aceton her und versetzt diese mit der berechneten Menge Salzsäure. Das isolierte und getrocknete farblose Kristallisat (Monohydrat) verliert bei erhöhter Temperatur und im Wasserstrahlvakuum vollständig seinen Wassergehalt und bildet gelbliche Kristalle von I mit dem Schmelzpunkt von 151 bis 152°C.

Sowohl die Imidazolidbildung wie auch die Glykolatumsetzung sind sehr wasserempfindlich und müssen deshalb unter absolut wasserfreien Bedingungen durchgeführt werden. Als Lösungsmittel werden Ether, wie Diisopropylether, Dioxan, Tetrahydrofuran (THF), Dimethoxyethan, vorzugsweise THF, verwendet. Die Reaktionstemperaturen liegen zwischen −10 bis 60°C, vorzugsweise für den ersten Schritt bei 0°C, beim zweiten bei 20°C.

2. Reaktionsschema: Synthese von I nach dem Imidazolid-Verfahren
(Stufen a–d)

a)

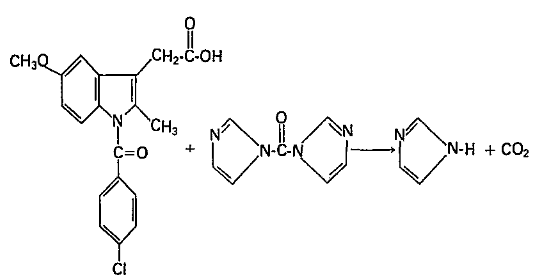

b)

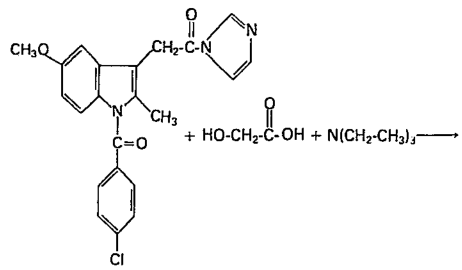

c)

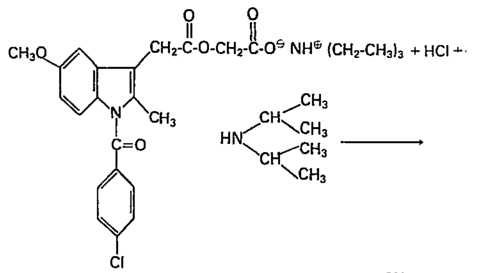

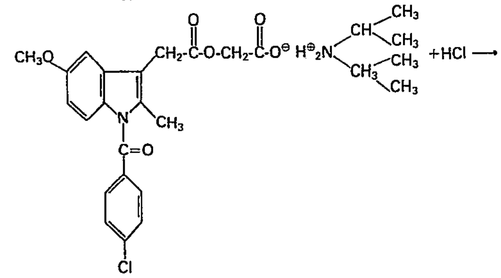

d)

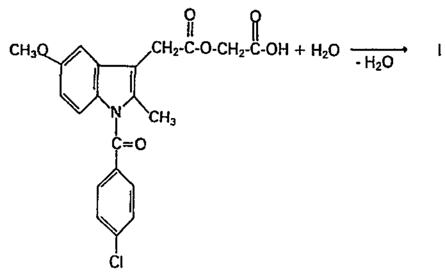

Beispiel 1

a) Imidazolid (II, $R^1$ - 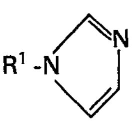 )

b) Umsetzung mit Triethylammoniumglykolat

c) Umwandlung in das Diisopropylammoniumsalz von I (Reaktionsfolge a–c ohne Isolierung der Zwischenprodukte)

10,8 g II ($R^1$ OH) (0,03 Mol) werden unter Rühren und Feuchtigkeitsausschluss bei 0°C in 70 ml abs. Tetrahydrofuran (THF) gelöst und mit 4,86 g Carbonyldiimidazol (0,03 Mol) versetzt. Man rührt 1 h bei Raumtemperatur nach und vervollständigt so die Kristallisation. Nun wird eine Lösung von 2,28 g Glykolsäure (0,03 Mol) und 4,14 ml Triethylamin (0,03 Mol) in 20 ml abs. Methylenchlorid ($CH_2Cl_2$) eingetropft, und man lässt 16 h die Reaktionsmischung unter Rühren bei Raumtemperatur nachreagieren. Zur Aufarbeitung destilliert man aus dem Reaktionsgemisch im Rotationsverdampfer unter Wasserstrahlvakuum bei 40°C das Lösungsmittel ab und versetzt den Rückstand mit 200 ml $CH_2Cl_2$. Die Methylenchloridlösung wird mit 90 ml 1n HCl versetzt und anschliessend dreimal mit je 100 ml $H_2O$ gewaschen. Nach Trocknen der Methylenchloridphase mit $Na_2SO_4$ wird die Lösung mit 4,2 ml Diisopropylamin (0,0292 Mol)

versetzt und im Rotationsverdampfer $CH_2Cl_2$ abdestilliert. Der Rückstand wird in 50 ml Aceton gelöst und nach Animpfen und Rühren (1 h bei Raumtemperatur) wird die kristalline farblose Substanz abgesaugt und bei 40°C im Exsikkator getrocknet.

Ausbeute: 6,8 g Diisopropylammoniumsalz von I.

d) Überführung in 1-(4-Chlorbenzoyl)-5-methoxy-2-methyl-3-indolacetoxyessigsäure I

Unter Rühren wird das so gewonnene Ammoniumsalz in 11 ml Aceton und 5 ml $H_2O$ gelöst. Anschliessend gibt man 8 ml 1n HCl zu dieser Lösung und impft an. Man rührt 1 h nach, bis die Kristallisation vollständig ist. Die farblose kristalline Substanz wird abgesaugt, mit $H_2O$ gut gewaschen und im Exsikkator bei 40°C im Wasserstrahlvakuum getrocknet.

Ausbeute: 4,6 g I-Hydrat = 35,4% d. Th.

Durch Trocknung bei 90°C im Wasserstrahlvakuum (1 h) entstehen unter vollständigem Wasserentzug gelbliche Kristalle von I, die bei 151–152°C schmelzen.

**Patentansprüche**

1. Verfahren zur Herstellung von 1-(4-Chlorbenzoyl)-5-methoxy-2-methyl-3-indolacetoxyessigsäure, dadurch gekennzeichnet, dass man das Derivat der Indolcarbonsäure der Formel

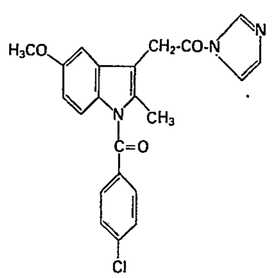

mit Verbindungen der Formel

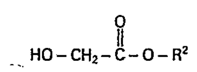

in welcher
$R^2$ für Wasserstoff oder Ammonium steht,
in Gegenwart von inerten organischen Lösungsmitteln unter absolut wasserfreien Bedingungen in einem Temperaturbereich von −10 bis 80°C umsetzt.

2. Verfahren nach Anspruch 1, dadurch gekennzeichnet, dass die Umsetzung in einem Temperaturbereich von −10 bis 50°C vorgenommen wird.

3. Verfahren nach Anspruch 1 oder 2, dadurch gekennzeichnet, dass als inerte organische Lösungsmittel Ether, chlorierte Kohlenwasserstoffe, substituierte Amide, Aromaten und/oder Ketone eingesetzt werden.

4. Verfahren nach den Ansprüchen 1 bis 3, dadurch gekennzeichnet, dass ein Ammoniumsalz der Glykolsäure eingesetzt wird.

**Claims**

1. Process for the preparation of 1-(4-chlorbenzoyl)-5-methoxy-2-methyl-3-indoleacetoxyacetic acid, characterised in that the derivative of indolecarboxylic acid of the formula

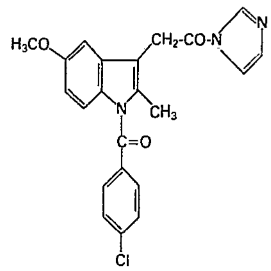

is reacted with compounds of the formula

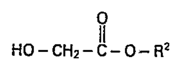

in which
$R^2$ represents hydrogen or ammonium,
in the presence of inert organic solvents under absolutely anhydrous conditions in a temperature range of −10 to 80°C.

2. Process according to claim 1, characterised in that the reaction is carried out in a temperature range of −10 to 50°C.

3. Process according to claim 1 or 2, characterised in that ethers, chlorinated hydrocarbons, substituted amides, aromatics and/or ketones are used as the inert organic solvents.

4. Process according to claims 1 to 3, characterised in that an ammonium salt of glycolic acid is used.

**Revendications**

1. Procédé de préparation de l'acide 1-(4-chlorobenzoyl)-5-méthoxy-2-méthyl-3-indolacétoxyacétique, caractérisé en ce que l'on fait réagir le dérivé de l'acide indole-carboxylique de formule

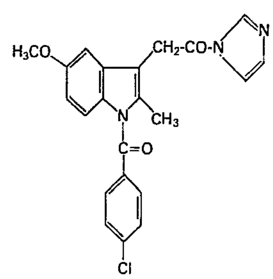

avec des composés de formule

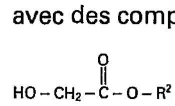

dans laquelle
$R^2$ représente l'hydrogène ou un groupe ammonium,
en présence de solvants organiques inertes et en milieu absolument anhydre dans l'intervalle de température de −10 à 80°C.

2. Procédé selon la revendication 1, caractérisé en ce que l'on effectue la réaction dans un intervalle de température de −10 à 50°C.

3. Procédé selon la revendication 1 ou 2, caractérisé en ce que l'on utilise en tant que solvants organiques inertes des éthers, des hydrocarbures chlorés, des amides substituée, des hydrocarbures aromatiques et/ou des cétones

4. Procédé selon les revendications 1 à 3, caractérisé en ce que l'on met en oeuvre un sel d'ammonium de l'acide glycolique.